# EUROPEAN PATENT APPLICATION

(11) **EP 3 715 368 A1**
(43) Date of publication of application: **30.09.2020**
(21) Application number: 19165672.7
(22) Date of filing: 28.03.2019
(51) Int. Cl.: C07K 14/705, C07K 14/725

(54) **CHIMERIC ANTIGEN RECEPTORS, VECTORS CODING FOR SUCH RECEPTORS AND THEIR USE IN THE MODIFICATION OF T CELLS**

(71) Applicant: Albert-Ludwigs-Universität Freiburg, 79085 Freiburg (DE)
(72) Inventor: SCHAMEL, Wolfgang, 79100 Freiburg (DE); MINGUET, Susanna, 79102 Freiburg (DE); HARTL, Frederike, 77977 Rust (DE)
(74) Representative: Keller, Günter

(57) **Abstract**

The present invention relates to a chimeric antigen receptor comprising at least the following components:
a) a peptidic structure capable of binding to a ligand;
b) an extracellular spacing structure;
c) a transmembrane domain;
d) none or at least one co-stimulatory domain; and
e) at least a TCR-derived activatory domain
whereby the TCR-derived activatory domain comprises the amino acid sequence RKGQRDLY (SEQ ID NO:1), which is capable to stimulate the lymphocyte specific Src kinase and its use in vectors for transducing T cells which are useful in the treatment of diseases, in particular tumors.

SEQUENCE LISTING

<110>  Albert-Ludwigs-Universität Freiburg

<120>  Chimeric Antigen Receptors, Vectors Coding for Such Receptors and
       their Use in the Modification of T cells

<130>  PEP02996A1b

<160>  6

<170>  PatentIn version 3.5

<210>  1
<211>  8
<212>  PRT
<213>  artificial sequence

<220>
<223>  binding to Scr kinase

<400>  1

Arg Lys Gly Gln Arg Asp Leu Tyr
1               5


<210>  2
<211>  8
<212>  PRT
<213>  artificial sequence

<220>
<223>  mutated binding region

<400>  2

Ala Ala Gly Gln Arg Asp Leu Tyr
1               5


<210>  3
<211>  5
<212>  PRT
<213>  artificial sequence

<220>
<223>  endoplasmatic reticulum retention signal

<400>  3

Asn Gln Arg Arg Ile
1               5


<210>  4
<211>  58
<212>  PRT
<213>  artificial sequence

<220>
<223>  murine derived pep

<400>  4

Tyr Trp Ser Lys Asn Arg Lys Ala Lys Ala Lys Pro Val Thr Arg Gly
1               5                   10                  15

Thr Gly Ala Gly Ala Arg Pro Arg Gly Gln Asn Lys Glu Arg Pro Pro
            20                  25                  30

Pro Val Pro Asn Pro Asp Tyr Glu Pro Ile Arg Lys Gly Gln Arg Asp
        35                  40                  45

Leu Tyr Ser Gly Leu Asn Gln Arg Ala Val
    50                  55

<210>  5
<211>  18
<212>  PRT
<213>  artificial sequence

<220>
<223>  murine derived pep

<400>  5

Asp Arg Glu Tyr Asp Gln Tyr Ser His Leu Gln Gly Asn Gln Leu Arg
1               5                   10                  15

Lys Lys

<210>  6
<211>  47
<212>  PRT
<213>  artificial sequence

<220>
<223>  murine derived pep

<400>  6

Gly His Glu Thr Gly Arg Pro Ser Gly Ala Ala Glu Val Gln Ala Leu
1               5                   10                  15

Leu Lys Asn Glu Gln Leu Tyr Gln Pro Leu Arg Asp Arg Glu Asp Thr
            20                  25                  30

Gln Tyr Ser Arg Leu Gly Gly Asn Trp Pro Arg Asn Lys Lys Ser
        35                  40                  45

## Description

Chimeric antigen receptors (CARs) are genetically constructed versatile synthetic receptors that provide T cells with a new and desired specificity. There is a therapeutic potential of T cells modified with a chimeric antigen receptor for the treatment of cancer, in particular in the treatment of B-cell malignancies. Currently, different CARs formats are available, such as the two FDA-approved anti-CD19 CARs (https://www.fda.gov/BiologicsBloodVaccines/CellularGeneTherapyProducts/ApprovedPro ducts/ucm573706.htm and https://www.fda.qov/NewsEvents/Newsroom/PressAnnouncements/ucm581216.htm), and more complex platforms such as the TRuC™ Platform (https://www.tcr2.com/our-approach/), bispecific chimeric antigen receptors or chimeric antigens using nanobodies (https://www.ncbi.nlm.nih.gov/pubmed/30672018).

The primary mediator of T cell activation is the T cell receptor (TCR). By replacing the naturally occurring antigen binding site with another binding site specific for a desired antigen a chimeric TCR can be generated. However, chimeric TCR must still recognize the tumor antigens associated to the major histocompatibility complex on the surface of tumor cells. This complex is often repressed by malignancies as mechanism of tumor scape. CARs, in contrast, recognize full proteins on the surface of tumor cells independently of the major histocompatibility complex. Human T cells, preferably CD4⁺ and/or in particular CD8⁺ T cells, can be genetically modified via transfection with a suitable vector coding for a CAR to redirect T cell specificity to the desired targets, e.g. proteins that are expressed in tumor cells.

Chang et al. (Trends in Molecular Medicine, May 2017, vol. 23, no. 5, pp 430-450) have described CARs as synthetic immuno-receptors for the treatment of cancer. Several structures of such chimeric antigen receptors are described and meanwhile several generations of CARs are known. The so-called first generation CARs consist of an extracellular antigen binding domain, an extracellular spacer, a transmembrane domain and a T cell activation domain, which normally is the CD3ζ domain. CD3ζ is a subunit of the T cell receptor (TCR) complex that must be phosphorylated to initiate T cell activation signaling networks. The second generation of CARs contains, in addition to the components described for the first generation CARs, a co-stimulatory domain from co-stimulatory receptors of T cells, such as 41BB or CD28. The third generation of CARs contains, in addition to the components of the second generation CARs, a second co-stimulatory domain.

It is one object of the present invention to provide alternative CAR designs, which have advantageous properties when used in the transfection of T cells. To date, most CAR designs have been overexpressed in the transfected T cells to obtain an efficient elimination of the tumor cells. However, overexpression of such CAR designs may have undesired properties as consequence of activation independently of target binding and/or non-physiological signal strengths (too much signal). These undesired properties included toxic side effects (cytokine release syndrome, CRS, as consequence of massive uncontrolled T cell activation) and/or inactivation of the transfected T cells by a process known as T cell exhaustion. Thus, an alternative strategy is, therefore, to specifically regulate the signaling properties of the CARs to make them more efficient.

The present invention discloses chimeric antigen receptors (CAR) which aims to improve the functionality comprising at least the following components:
a) a peptidic structure capable of binding to a ligand;
b) an extracellular spacing structure;
c) a transmembrane domain;
d) none or one co-stimulatory domain; and
e) at least one TCR-derived activatory domains.

The at least one complementary TCR-derived activatory domain includes in preferred embodiments the amino acid sequence RKGQRDLY (SEQ ID NO:1), and, when indicated, excludes the amino acid sequence NQRRI (endoplasmic reticulum retention signal, SEQ ID NO:3). The amino acid sequence RKGQRDLY (SEQ ID NO:1) is capable to bind the lymphocyte specific Src kinase.

A decisive part for the specific function of the CAR is the peptidic structure capable of binding to a ligand. The purpose of this part of CAR is to bring the modified T cell in close connection to the target cell (tumor cell) whereby the peptidic structure can bind sufficiently specific and with sufficient strength to the target molecule. The target molecule should be a specific structure, which is expressed (ideally) only at the target cell, which should be destroyed. Such a ligand can be a specific tumor antigen. There are many different tumor antigens known like for example α-feto-protein, carcinoembryonic antigen, epithelial tumor antigen, PSA or PSMA to name only a few. In most cases, however, these tumor antigens are also expressed in subpopulations of healthy cells. It is important therefore, that the healthy cells that are going to be co-targeted are not vital for the patient.

The ligand to which the peptidic structure binds may also be a specific antigen expressed at the surface of cells infected with viruses, bacteria or other organisms which cause diseases in humans and which are difficult to treat, because the foreign disease causing agent lives and propagates with cells of the host.

Methods to generate peptidic structures which bind to such ligands are well-known. The probably most commonly known method is the generation of monoclonal antibodies with the help of mice. When a suitable monoclonal antibody has been identified, the amino acid sequences can be obtained. Such monoclonal antibodies or the antigen binding parts thereof may be genetically manipulated to obtain binding structures which exhibit a strong specific binding to the target sequence and which do preferably not contain foreign amino acid sequences which may induce undesired immunological effects. Such peptidic sequence can for example be humanized. A well-known form of such peptidic structures are scFv structures which are used in preferred embodiments of the present invention.

The antigen binding part (scFv structures) and the transmembrane region are usually linked by a hinge region. The purpose of this hinge region is to bring the antigen binding region in a suitable steric position and distance to the cell membrane.

The extracellular spacing structure is intended to bring the structure which binds to the ligand in a correct sterical orientation apart from the cytoplasma membrane of the T cell. Such extracellular spacing structure may be derived from receptors of T cells or it may also
be an artificial structure like a polyglycine linker having for example 5-10 amino acids or combinations thereof. Other structures and combinations of such structures are also useful.

The transmembrane domain is usually obtained from a T cell receptor and stretches through the cytoplasmic membrane. The transmembrane domain helps to embed the CAR into the cytoplasma membrane of the T cell.

A CAR can, according to the present invention, have none, one or more, such as two or three co-stimulatory domains. The CAR used in the present invention as proof of principle contains one co-stimulatory domain, which is derived from the 41BB receptor. The main function of this co-stimulatory domain is to provide additional signals to the T cell for optimal T cell activation. In particular, the 41BB co-stimulatory domain seems to prolong the persistence of CAR T cells in individuals upon injection allowing a better elimination of the tumor cells.

According to the present invention, the preferred CAR has **two** TCR-derived activatory domains derived from the T cell receptor (TCR). In particular, domains derived from the CD3ζ chain and from the CD3ε chain. It is an essential feature of the present invention that the activatory domain derived from the CD3ε chain comprises the amino acid sequence RKGQRDLY (SEQ ID NO:1) and might exclude the amino acid sequence NQRRI (endoplasmic reticulum retention signal).

The chimeric antigen receptor (CAR) is provided by inserting the genetic information coding for such chimeric antigen receptor into a suitable vector. Such vector may be a plasmid or a vector derived from a virus preferably a lentiviral vector. The CAR encoded by a lentiviral vector may thus be used for the transfection of T cells. In a preferred embodiment of the present invention, the T cells of a patient to be treated are isolated ex vivo by a suitable leukapheresis proceeding. Such T cells are in vitro transfected with the suitable vector, preferably the lentiviral vector, and the transfected cells are isolated and amplified in culture. After the amplification of the genetically modified T cells, such cells are applied to a patient wherein the cells can act and kill the undesired cells. The T cells bind to the target cells (e.g. tumor cells) and are killing them.

The CARs according to the present invention are therefore suitable for use in the treatment of diseases. Such diseases are in particular various forms of cancer. The CARs according to the present invention may also be used in the treatment of other diseases, which are difficult to treat like chronic infections or the treatment of severe viral infections. Likewise, more complex platforms such as the "T-cell receptor (TCR) fusion protein" or "TFP" Platform could be used for similar pathological scenarios. A"TFP" as referred to herein includes a recombinant polypeptide derived from the various polypeptides comprising the TCR that is generally capable of i) binding to a surface antigen on target cells and ii) interacting with other polypeptide components of the intact TCR complex, typically when co-located in or on the surface of a T-cell. A "TFP T Cell," as used herein, is defined as an immune cell comprising a T cell receptor complex wherein one or more TCR or CD3 subunits has been engineered to include a binding moiety, e.g., an antibody or fragment thereof. In some embodiments, the antibody or fragment thereof comprises an scFv. In other embodiments, the antibody or fragment thereof comprises a single domain antibody (sdAb), e.g., a camelid or fragment thereof. The engineered subunit comprises (1) at least a portion of a TCR extracellular domain, (2) a transmembrane domain, and (3) an intracellular domain comprising a stimulatory domain from an intracellular signaling domain of CD3 epsilon, CD3 gamma, CD3 delta, TCR alpha or TCR beta (or, alternately, TCR gamma or TCR delta), and a sequence encoding a TCR constant domain, wherein the TCR constant domain is a TCR alpha constant domain, a TCR beta constant domain or a TCR alpha constant domain and a TCR beta constant domain; wherein the TCR subunit and the antigen domain are operatively linked, and wherein the TFP functionally incorporates into a TCR complex when expressed in a T cell. The TFP T cell platform is described in International Patent Publication Nos. WO2018098365, WO2016187349, WO2018067993, and WO2018026953.

Such modified T cells having a CAR exhibit highly desirable properties. The antigen-recognition domain is preferably a single-chain variable fragment (scFv) which can be derived from a suitable monoclonal antibody and which may preferably target a tumor-associated antigen. This domain can be, for example derived from an antibody recognizing CD19 in the context of a treatment of B-cell malignances such as lymphoma. It can, however, also bind to other antigens which are specific for tumor cells like PSA or PSMA for prostate cancer. The term "antigen-recognition domain" comprises also modified antigen binding sequences, in particular when the sequence is derived from mouse monoclonal antibodies. For the application to humans such sequences are preferably humanized in order to avoid unspecific immune reactions against sequences which are recognized by the human immune system as foreign (mouse).

Without wishing to be bound to a theory it is assumed that the mechanism on which invention is based has to do with a non-canonical binding of the LCK SH3 domain to CD3ε which redefines initiation of TCR signaling.

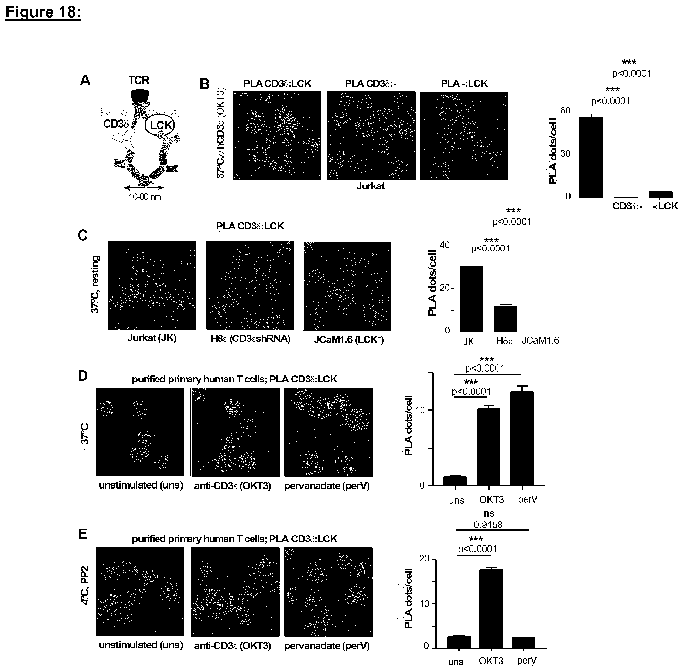

Several experiments have been performed to elucidate the mechanism of TCR signal initiation in T cells. The results of such experiments are summarized below and in particular in Figures 18-24.

### Functional considerations:

The T cell antigen receptor (TCR) is a multimeric protein complex expressed on the surface of T cells. The αβ TCR is composed of the ligand-binding TCRαβ heterodimer and the signal-transducing CD3 complex. In contrast to TCRαβ, CD3 subunits contain immunoreceptor tyrosine-based activation motifs (ITAMs) in their cytoplasmic tails that mediate signal transduction. Upon TCR binding to its ligand (major histocompatibility complex bound to an antigenic peptide, MHCp), the ITAMs are phosphorylated on tyrosine residues by the sarcoma (SRC) family kinase, LCK, and TCR signaling is initiated. In the absence of this phosphorylation, T cells do not get activated.

Lymphocyte specific Src kinase (LCK) is a modular protein, which plays an important role in the present invention. An acylated N-terminal part attaches LCK to the plasma membrane. A unique domain mediates LCK binding to the CD4 and CD8 coreceptors. An SH3 domain mediates binding to proline-rich sequences, whose basic sequence is PxxP, and an SH2 domain mediates phosphotyrosine interactions. LCK harbors a kinase domain containing the activating tyrosine, Y394, and a short C-terminal regulatory tail containing the inhibitory tyrosine, Y505.

LCK catalytic activity is inhibited by cooperative, intramolecular interactions between the SH3 domain and a proline-rich sequence situated in the linker between the SH2 and kinase domains, and between the SH2 domain and the phosphorylated Y505. This autoinhibited kinase is a precariously set mousetrap, in which alternative interactions can be sufficient for activation. SH3 domain displacement via binding to PxxP sequences in the CD28 coreceptor, the UNC119 adaptor protein or the viral proteins, Tip and Nef, activates LCK. Phosphorylation of Y394 is the strongest activator of LCK catalytic activity.

A mechanistic explanation of how LCK is recruited to the ligated TCR to initiate CD3 phosphorylation remains elusive. Among the existing models, it has been proposed that the formation of a trimolecular complex of the TCR with MHCp and the CD4 or CD8 coreceptors brings coreceptor-associated LCK molecules close to the CD3 cytoplasmic tails to initiate phosphorylation. However, recent reports show that complex formation occurs in two stages. The MHCp ligand first binds the TCR and results in CD3 ITAM phosphorylation by a free, rather than coreceptor-bound, pool of LCK. The subsequent MHCp-coreceptor binding recruits the coreceptor-associated LCK via interaction between the LCK SH2 domain and the phosphorylated ITAMs, and increases T cell sensitivity. That the proportion of Y394-phosphorylated LCK remains constant upon ligand binding to the TCR supports an alternative triggering hypothesis, which proposes that spatial reorganization of LCK within the plasma membrane might play an important role in TCR signaling. Other mechanisms for TCR triggering have also been proposed. Segregation of phosphatases from the vicinity of ligated TCRs might favor accumulation of phosphorylated ITAMs. TCR phosphorylation might also be controlled by conformational changes within the receptor regulating the accessibility of the CD3 ITAMs to LCK. While these models theorize mechanistic steps for ligand triggering of TCR signaling, they say nothing about how LCK is targeted to the TCR to initiate CD3 phosphorylation.

T cell-based immunotherapy using chimeric antigen receptors (CARs) is a major breakthrough in the treatment of cancer providing hope for curative responses in patients with hematological malignances. CARs are synthetic receptors redirecting T cells to mediate tumor rejection by combining signaling domains from the TCR (namely the cytoplasmic tail of the ζ chain) and from co-stimulatory receptors (such as 41BB). ITAM phosphorylation is also crucial for signal initiation of CARs. How LCK phosphorylates the ITAMs of these chimeric receptors remains unknown.

Without wishing to be bound to a theory, the chimeric receptor according to the present invention rationally integrates the domain that recruits LCK to phosphorylate the ITAMs to a chimeric receptor to improve anti-tumor functionality of chimeric antigen receptor (CAR).

### Experimental Results:

In the course of the present invention, it was investigated whether LCK localization in respect to the TCR changes upon ligand binding to the TCR. The proximity of the CD3δ cytoplasmic tail to LCK was analyzed in intact cells. The *in situ* proximity ligation assay was used, which lacks the drawbacks of detergent lysis. The presence of a fluorescent dot indicates that the distance between one CD3δ and one LCK protein is less than 80 nm. Several controls demonstrated the specificity of this approach. In resting T cells, TCR:LCK interactions were above background readings in the assay, indicating that a detectable proportion of LCK molecules are near the TCR. To investigate whether this proximity changes upon TCR stimulation, the assay was performed before and after TCR stimulation at 37°C. Anti-human CD3ε (anti-CD3ε, OKT3) antibody stimulation was used to solely focus on the proximity between LCK and TCR without involving other LCK-interacting proteins such as CD4, CD8 or CD28. The number of LCK molecules in close proximity to TCR molecules increased upon stimulation in primary human T cells. Similar results were obtained in concert with pervanadate treatment, which inhibits phosphatases inducing TCR phosphorylation. Stimulation with anti-CD3 antibodies at 37°C results in ITAMs phosphorylation, meaning that the LCK SH2 domain could have bound to the phosphorylated ITAMs to achieve the proximity shift. The TCR lacks intrinsic kinase activity, and cannot phosphorylate itself to generate the first docking site for the LCK SH2 domain. It is assumed that LCK recruitment to the TCR must, therefore, precede any phosphorylation event. To mimic phosphorylation-independent conditions during analysis of CD3δ:LCK proximity, analyses were performed in the presence of the SRC kinase inhibitor, PP2. Strikingly, the number of TCR:LCK interactions significantly increased upon stimulation with anti-CD3ε in primary human T cells. Antibody binding mimics antigen binding and results in a reversible conformational change in the CD3 cytoplasmic tails (CD3CC) that stabilizes the *active* TCR conformation prior to ITAM phopsphorylation. Treatment with pervanadate at 4°C in the presence of PP2 served as a control in which neither the *active* TCR conformation is stabilized nor ITAM phosphorylation takes place. The extent of basal TCR:LCK interactions upon pervanadate treatment was similar to unstimulated samples, demonstrating that the tests were indeed performed under SRC kinase phosphorylation-free conditions. The data demonstrate that LCK is recruited to *active* TCRs before ITAM phosphorylation occurs **(****Figure 18****).**

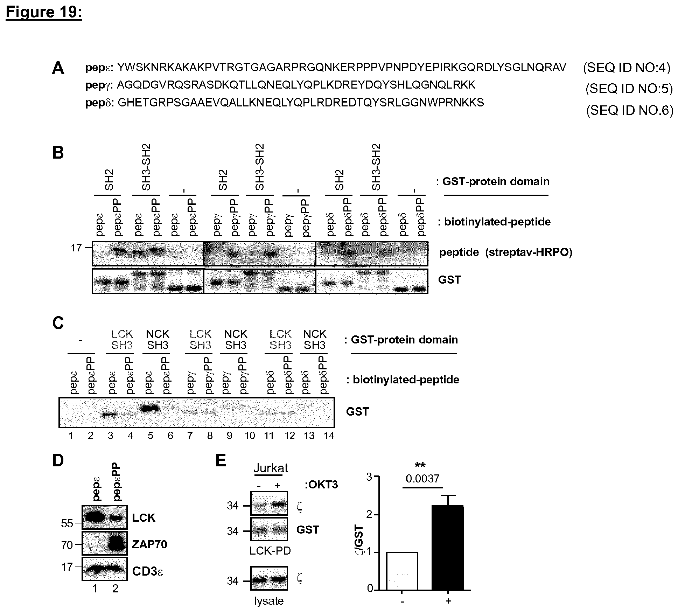

To identify the LCK protein domain responsible for binding to the non-phosphorylated TCR in the *active* TCR conformation, associations were tested between either LCK SH2 or SH3 domains and peptides containing the complete CD3 cytoplasmic tails either with or without ITAM phosphorylation. Consistent with the pervanadate-derived results, the LCK SH2 bound to the phosphorylated CD3ε, CD3γ and CD3δ peptides. A fusion protein containing both the LCK SH3 and SH2 domains also bound to non-phosphorylated CD3ε, but not to non-phosphorylated CD3γ and CD3δ peptides. SH2 domains bind to phosphorylated tyrosines, and therefore, they cannot mediate the observed binding to non-phosphorylated CD3ε. Hence, it was tested whether the LCK SH3 domain alone can bind to the CD3 cytoplasmic tail. The NCK SH3.1 domain was used, which binds to the CD3ε proline-rich sequence, as a positive control. Indeed, the LCK SH3 domain bound to the non phosphorylated CD3ε cytoplasmic tail, suggesting that this interaction mediates LCK recruitment to the TCR. Incubation of Jurkat T cell lysates with phosphorylated and non-phosphorylated CD3ε peptides showed that endogenous full length LCK bound both phosphorylated and non-phosphorylated CD3ε peptides. As expected, ZAP70 bound only the phosphorylated CD3ε peptide. Lysates from unstimulated Jurkat T cells or Jurkat T cells stimulated with anti-CD3ε were next incubated with LCK SH3 domain-coated beads. Antibody stimulation resulted in increased TCR binding to LCK SH3. Altogether, these data indicate that the LCK SH3 domain binds to the non-phosphorylated CD3ε cytoplasmic tail upon TCR stimulation **(****Figure 19****).**

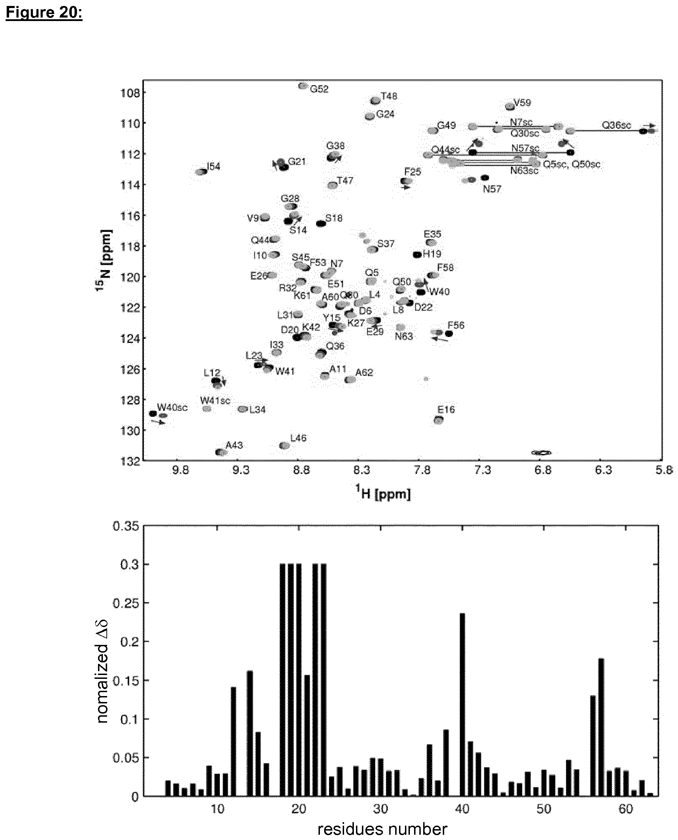

To gain insights into the molecular details of the LCK SH3 interaction with CD3ε, nuclear magnetic resonance (NMR) spectroscopy was used. The LCK SH3 domain and the CD3ε cytoplasmic tail were expressed in *E. coli.* Spectral changes of 15N-labeled LCK SH3 were observed in a series of 1H, 15N heteronuclear single-quantum correlation experiments (HSQC) with addition of the unlabeled CD3ε cytoplasmic tail in molar excess. Prominent
signal changes were observed for the S18-L23, W40 and F56-N57 residues in the LCK SH3. These chemical shift changes conclusively demonstrate an interaction between LCK SH3 and CD3ε **(****Figure 20****).**

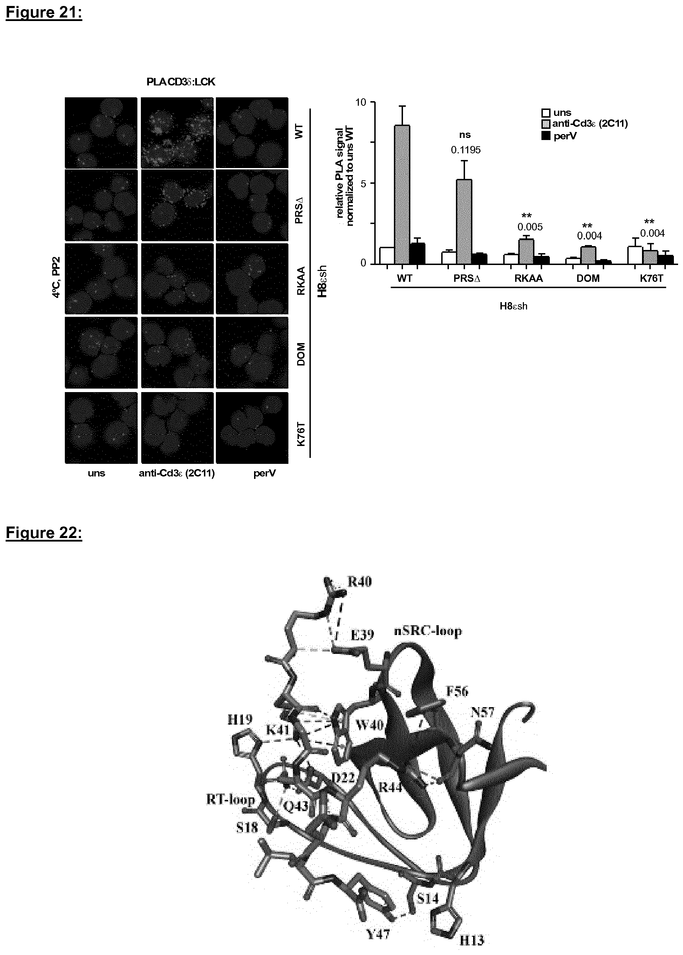

SH3 domains bind to proline-rich sequences, and CD3ε contains the proline-rich sequence (PRS), PxxPxxDY, which is only accessible in the *active* TCR conformation. To investigate whether LCK binds PxxPxxDY, Jurkat-derived, CD3ε-silenced cell lines (named H8εsh cells) were generated expressing murine wildtype (WT) Cd3ε, mutant Cd3ε containing the proline-depleted AxxAxxDY motif (PRSΔ), which was described to abolish SH3 NCK binding, or Cd3ε with the K76T mutation, which prevents the TCR from switching to its *active* conformation. It was first confirmed that WT and mutant Cd3ε molecules are expressed and behave as expected once expressed in the cells (such data are not shown in a Figure). These new cell lines allowed to test whether the CD3ε PxxPxxDY binds to LCK in the absence of ITAM phosphorylation. Only the introduced murine Cd3 molecules were stimulated by incubating the cells with an anti-Cd3ε antibody (145-2C11) at 4°C in the presence of PP2, and the CD3δ:LCK proximity ligation assay was performed. Unexpectedly, TCR:LCK interactions increased similarly in both H8εsh cells expressing Cd3εPRSΔ or Cd3εWT upon antibody-mediated stabilization of the *active* TCR conformation. Contrastingly, hardly any proximity was detected between the TCR and LCK in H8εsh cells expressing Cd3εK76T. Taken together, these data demonstrate that the TCR in its *active* conformation exposes a yet unidentified LCK binding site in CD3ε **(****Figure 21****).**

Although most SH3 domains bind the core PxxP motif, some exceptions have been reported. The FYB SH3 domain binds the proline independent RKxx(Y)xxY motif in the SKAP1 adaptor protein, to which the SH3 domains of FYN and LCK bind with 10-fold less affinity. The related RKGQRDLY (SEQ ID NO:1) sequence was identified, which is conserved from lobe-finned fishes to mammals, in the CD3ε cytoplasmic tail. To investigate whether this sequence might interact with the LCK SH3 domain, RK was mutated to AA while leaving the rest of the sequence intact: AAGQRDLY (SEQ ID NO:2). The mutated Cd3ε (Cd3εRKAA) was expressed in H8εsh cells, and performed the proximity ligation assay under phosphorylation-free conditions. A double mutant combining RKAA and PRSΔ mutations was also created (Cd3εDOM). All cell lines were characterized. In contrast to the cells expressing WT Cd3ε or Cd3εPRSΔ, hardly any proximity between the TCR and LCK was detected in H8εsh cells expressing Cd3εRKAA or Cd3εDOM. Taken together, these data indicate that the ligated TCR exposes the RK sequence, which mediates LCK SH3 domain binding and, thereby, LCK recruitment to the TCR prior to any ITAM phosphorylation **(****Figure 21****).**

To gain molecular insights into this non-canonical interaction, a protein-protein docking simulation was performed with the CD3ε cytoplasmic tail and the LCK SH3 domain as input structures using the HADDOCK docking web server. The RKGQRDLY (SEQ ID NO:1) sequence in CD3ε and L12, S14, S18, H19, D20, G21, D22, L23, W40, F56, and N57 residues in the LCK SH3 domain were used as ambiguous interaction restraints. The top-ranked model for docking showed that the CD3ε R40, K41, Q43, R44, and Y47 residues interacted with H13, S14, S18, H19, D22, E39, W40, F56, and N57 in LCK SH3. R40 interacted with E39 via a hydrogen bond and an electrostatic interaction. K41 mediated a salt-bridge interaction with D22, hydrogen bond interactions with H19, D22, and W40, and a network of hydrophobic and electrostatics interactions with W40. These anchorages of the LCK SH3 domain in the RT and n-SRC loops support a critical role of the CD3ε R40 and K41 residues in LCK SH3 binding to CD3ε. Q43 interacts with S18 and D22 *via* hydrogen bonds, R44 interacts with F56 via a π-cation interaction and with N57 via a hydrogen bond and Y47 forms a hydrogen bond with S41. This Y47-S41 interaction using the CD3ε Y47 hydroxyl group is abolished upon Y47 phosphorylation, explaining why the LCK SH3 domain failed to bind the phosphorylated CD3ε peptide. We speculate that Y47 phosphorylation might mechanistically explain the transient interaction of LCK to the ligand-bound TCR. Altogether, these results show that the RKGQRDLY (SEQ ID NO:1) sequence (now called the Receptor Kinase interaction or RK motif) in CD3ε binds to the LCK SH3 domain **(****Figure 22****).**

To investigate whether LCK binding to the RKGQRDLY (SEQ ID NO:1) sequence in CD3ε increases LCK activity at the TCR, a reporter for LCK activity was designed that is only phosphorylated if active LCK is in the vicinity of the TCR. This reporter consists of an inactive LCK kinase domain (K273A mutant) coupled to the CD3ζ chain by a flexible linker, and fulfills the following criteria:
(i) the K273A mutation keeps the LCK kinase domain enzymatically inactive,
(ii) Y394 in the LCK kinase domain is a well characterized substrate for LCK, whose phosphorylated form can be detected with specific antibodies and
(iii) the LCK kinase domain is constitutively exposed.

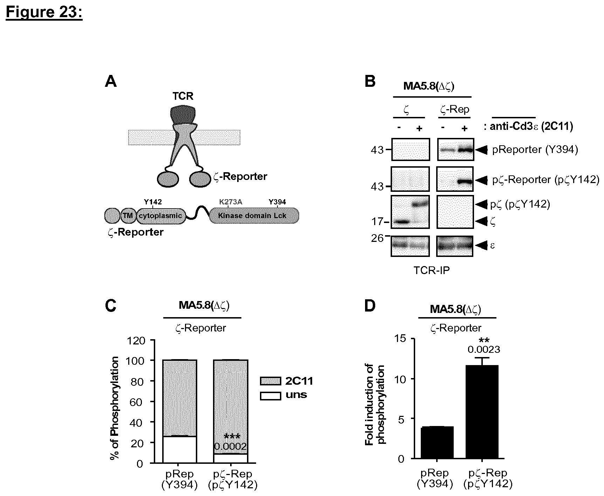

This reporter (referred to as ζ-Reporter) was expressed in ζ-deficient MA5.8 murine T cells, and cells were analyzed for Cd3 ITAM and reporter phosphorylation. In unstimulated T cells, phosphorylation of the ζ-Reporter at the kinase-dead domain (pY394) was readily detectable, suggesting that active LCK is in close proximity to the TCR in resting T cells. Despite that, hardly any phosphorylation at the ζ ITAMs (pζY142) was observed, indicating that in the resting TCR, the ITAMs are protected from phosphorylation. Phosphorylation of Y394 in the ζ-Reporter, was inhibited by PP2, supporting that LCK was the phosphorylating kinase (data not shown). On the one hand, TCR stimulation resulted in a 4-fold increase of Y394 phosphorylation of the ζ-Reporter despite being constantly accessible. These data demonstrate that LCK activity at the TCR increased upon TCR activation, most probably by LCK recruitment to the RK motif in the ligated TCRs. On the other hand, TCR stimulation resulted in a larger increase in ζ ITAM phosphorylation, namely 11-fold. These data suggest that a pool of active LCK is close to the resting TCR, in which the ITAMs are protected from spurious phosphorylation. In conclusion, antibody-mediated stabilization of the *active* TCR conformation leads to a local increase of LCK activity at the TCR and to the exposure of the ITAMs for phosphorylation **(****Figure 23****).**

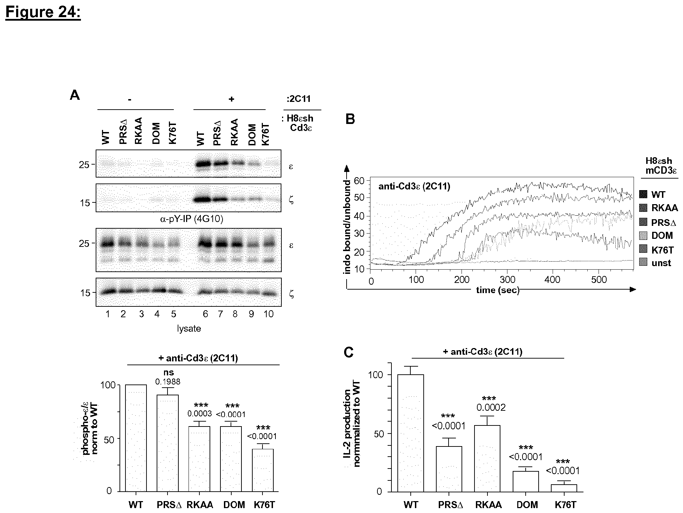

To test a functional implication of the LCK SH3 domain recruitment to the RK motif in T cell activation, the CD3δ:LCK proximity ligation assay was performed under more physiological conditions, namely at 37°C in the absence of kinase inhibition. In Cd3εWT- or Cd3εPRSΔ-expressing cells, the basal TCR:LCK proximity increased after stimulating both with antibodies or pervanadate. In contrast, hardly any TCR:LCK interaction was observed in cells expressing the Cd3RKAA mutant or the CD3CC-deficient TCR. Next, the role of the RK motif in CD3 phosphorylation was investigated. Cells reconstituted with Cd3εWT showed strong TCR phosphorylation upon stimulation. In contrast, TCR phosphorylation was reduced by 40% in Cd3εRKAA- and in Cd3εDOM-expressing cells compared to Cd3εWT cells. TCR phosphorylation was decreased by 60% in CD3CC-deficient cells. These data indicate that the RK motif is required to initiate optimal TCR phosphorylation. The influence of the RK motif on downstream TCR signaling was also tested, such as calcium influx and IL2 production. Mutation of the RK motif resulted in a delayed and decreased calcium response upon antibody stimulation. Cells expressing Cd3εPRSΔ exhibited reduced TCR-induced calcium influx, most likely because the interaction of the TCR with NCK was prevented. Expression of Cd3εDOM and Cd3εK76T caused a further reduction and delay of calcium influx. Finally, mutation of the RK or proline-rich sequence motifs caused reduced IL2 production upon stimulation with anti-Cd3ε and anti-Cd28 antibodies. This reduction was enhanced in the Cd3εDOM and in CD3CC-deficient cells. Taken together, these data indicate that the RK motif plays an important role in stimulation-induced phosphorylation of the CD3 cytoplasmic tails impacting downstream TCR signaling **(****Figure 24****).**

An important aspect of the present invention is that the RK motif (SEQ ID NO:1) is essential for efficient signaling in antigen-induced TCR activation.

In the present invention is shown how LCK is targeted to the TCR, and this is used in a new concept of signal initiation. LCK is recruited to the ligated TCR by direct binding of the LCK SH3 domain to the CD3ε cytoplasmic tail prior to receptor phosphorylation. SH3 domains commonly bind to PxxP sequences, and CD3ε proline-rich sequence exposure is the best-characterized hallmark of the ligand-bound TCR. LCK recruitment occurred despite mutation of the proline-rich sequence in the experiments. NMR spectroscopy and molecular modelling demonstrated that the non-canonical SH3-binding motif, the RKGQRDLY (SEQ ID NO:1) sequence, in the CD3ε cytoplasmic tail, which is termed the RK motif, binds to both the RT-loop and n-SRC loop of the LCK SH3 domain. Most LCK RT-loop residues are not conserved in FYN or other SRC family kinases, possibly explaining why FYN cannot substitute for LCK during T cell development and activation. Two related RK motifs have been identified, one in the SKAP1 adaptor protein (RKxx(Y)xxY), and another one in *C.albicans.* These are the first proline-independent SH3 binding motifs described. Their distribution across the evolutionary panorama suggests that SH3 domains may bind to non-canonical motifs more often than previously thought, redefining the one-key-for-one-lock view of protein-protein interaction.

RK motif mutation (sequence AAGQRDLY, SEQ ID NO:2) prevented LCK binding to the TCR and reduced antibody-induced CD3 phosphorylation, calcium mobilization, and IL2 production involved with downstream signaling **(****Figure 24****).** Similarly, overexpressing a peptide containing the related SKAP1 RK motif in murine splenocytes and human T cell lines attenuated *Il2* and *IL2* transcription, respectively. However, TCR signaling was only completely blocked in cells expressing CD3CC-deficient or doubly mutated receptors, suggesting that CD3CC-mediated exposure of both the proline-rich sequence and RK motif is needed for CD3 phosphorylation. NCK1 recruitment to the proline-rich sequence has been shown to be important for TCR phosphorylation and downstream signaling, and LCK recruitment was hindered in cells expressing a mutated RK motif. Taken together, these data provide a mechanistic explanation to the complete block of receptor signaling observed in CD3CC-deficient TCRs, since these mutant receptors expose neither the proline-rich sequence nor the RK motif and recruit neither NCK1 nor LCK.

A proportion of LCK is active in resting T cells. It is assumed that LCK is close to the TCR in resting cells, whether this LCK is actually active, was unclear. For this reason, a novel reporter for LCK activity was developed to demonstrate that active LCK is in close proximity to the TCR in resting cells. This local LCK activity further increased 4-fold upon receptor stimulation. The individual contributions of LCK recruitment to the TCR (as detected by proximity ligation assay), of conformational changes of the recruited LCK molecules and of exclusion of phosphatases to the local enhancement of LCK activity should be investigated further. Conversely, phosphorylation of the CD3 ITAMs increased 11-fold, suggesting that other mechanisms beyond the increase in local LCK activity foster ITAM phosphorylation. These mechanisms might be TCR intrinsic, such as regulation of ITAM accessibility either by stabilization of the *active* TCR conformation or release of the CD3 cytoplasmic tails from the plasma membrane.

Altogether, the work forming the basis of the present invention summarized how phosphorylation of the TCR is initiated:
(i) ligand binding stabilizes the T cell receptor (TCR) in its *active* conformation in which the RK motif (sequence RKGQRDLY, SEQ ID NO:1) and the CD3 ITAMs become accessible;
(ii) the LCK SH3 domain binds the RK motif, recruiting LCK to the TCR, and
(iii) recruited LCK phosphorylates the ITAMs.

The results clearly demonstrate that binding of LCK directly to the RK motif (sequence RKGQRDLY, SEQ ID NO:1) triggers TCR signal initiation. Consequently this sequence is preferably contained within the constructs of the invention.

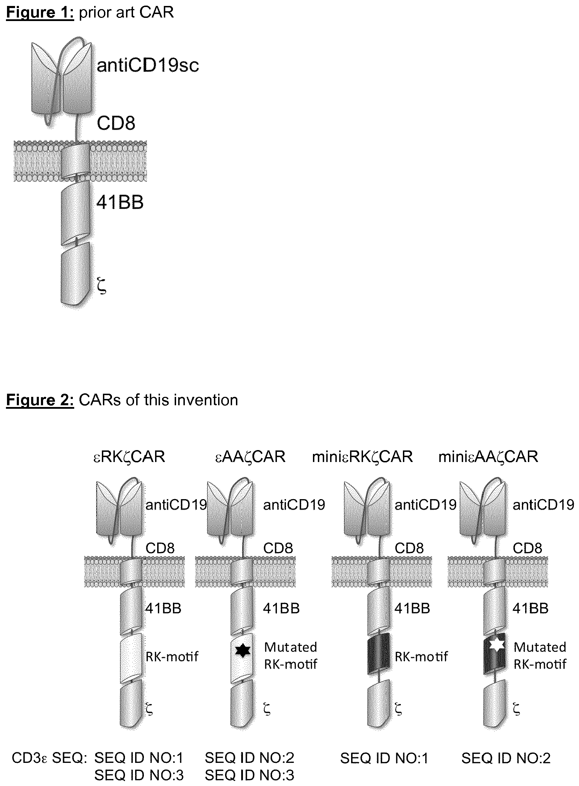

According to the present invention, the RK motif (sequence RKGQRDLY, SEQ ID NO:1) was introduced in a well-characterized anti-CD19 CAR (from now on called ζCAR, Figure 1). The starting construct was described by Imai et al., Leukemia, 2004, 18, 676-684, and Maude et al., N.Engl.J.Med. 2018, 378, 439-448. We aim, to analyze the impact of LCK recruitment to chimeric receptors on the anti-tumor functionality of T cells. This approach serves as proof-of-principle of the applicability of adding a complementary TCR-derived activatory domain include in preferred embodiments the amino acid sequence RKGQRDLY (SEQ ID NO:1).

Four chimeric antigen receptors were designed (Figure 2), which are based on the well-characterized anti-CD19 CAR (here named called ζCAR, Figure 1, (Imai et al., 2004; Maude et al., 2018)).:
1. By adding part of the CD3ε cytoplasmic tail including the RK motif (sequence RKGQRDLY, SEQ ID NO:1) between the 41BB and ζ sequences. This chimeric receptor will be named εRKζCAR.
2. By adding part of the CD3ε cytoplasmic tail including a mutated RK motif (sequence AAGQRDLY, SEQ ID NO:2) between the 41BB and ζ sequences. This chimeric receptor will be named εAAζCAR.
3. By adding part of the CD3ε cytoplasmic tail including the RK motif (sequence RKGQRDLY, SEQ ID NO:1) and excluding the endoplasmatic reticulum retention signal having the amino acid sequence NQRRI (SEQ ID NO:3). This chimeric receptor will be named miniεRKζCAR.
4. By adding part of the CD3ε cytoplasmic tail including a mutated RK motif (sequence AAGQRDLY, SEQ ID NO:2) between the 41BB and ζ sequences and excluding the endoplasmatic reticulum retention signal having the amino acid sequence NQRRI (SEQ ID NO:3). This chimeric receptor will be named miniεAAζCAR.

These four chimeric antigen receptors were used in the present invention and compared to the well-characterized anti-CD19 CAR (here named called ζCAR, Figure 1, (Imai et al., 2004; Maude et al., 2018)). The results are described in more detail in the following Figures (1-17).

In the course of the present invention several experiments have been performed and the results are shown in the Figures 1-17. The details of the experiments and the Figures relate to the present invention unless expressly stated. Important aspects of the present invention are summarized in the following Tables:

**Table 1: The sequences used in this invention**

| **SEQ ID NO:** | **Amino acid sequence** | **Origin / Comments** |
|---|---|---|
| 1 | RKGQRDLY | Human CD3ε / here named RK motif |
| 2 | AAGQRDLY | Mutation of SEQ ID NO:1 |
| 3 | NQRRI | Human CD3ε (ER retention signal) |

**Table 2: Correlation between Figures and experiments**

| **Figure:** | **Title/Description** | **Related to Experiment** |
|---|---|---|
| 1 | prior art CAR | All |
| 2 | CARs of this invention (with controls) | All |
| 3 | Constructs coding for the CARs used in the present invention | All |
| 4 | Level of CAR surface expression in CAR T cells | Experiment 1 |
| 5 | Cytotoxicity assay; Target cell Nalm6 | Experiment 2 |
| 6 | Cytotoxicity assay; Target cell DAUDI | Experiment 3 |
| 7 | Cytotoxicity assay; Target cell Nalm6 (Alternative analysis) | Experiment 2 |
| 8 | Cytotoxicity assay; Target cell Nalm6 (Alternative analysis) | Experiment 2 |
| 9 | Cytotoxicity assay; Target cell DAUDI (Alternative analysis) | Experiment 3 |
| 10 | Cytotoxicity assay; Target cell DAUDI (Alternative analysis) | Experiment 3 |
| 11 | Cytotoxicity assay; Target cell Nalm6 (Alternative analysis) | Experiment 2 |
| 12 | Cytotoxicity assay; Target cell Nalm6 (Alternative analysis) | Experiment 2 |
| 13 | Cytotoxicity assay; Target cell DAUDI (Alternative analysis) | Experiment 3 |
| 14 | Exhaustion markers in the absence of target cells | Experiment 4 |
| 15 | Time line of the pre-clinical model | Experiments 5 and 6 |
| 16 | Stress-test *in vivo* | Experiment 5 |
| 17 | Pre-clinical model *in vivo* | Experiment 6 |

### Figures supporting and disclosing the present invention:

**Figure 1** shows schematically a chimeric antigen receptor (CAR), which received FDA approval (prior art) (https://www.novartis.com/news/media-releases/kymriahr-tisagenlecleucel-first-class-car-t-therapy-from-novartis-receives-second-fda-approval-treat-appropriate-rr-patients-large-b-cell-lymphoma). This CAR has the following components: An antiCD19sc part as an antigen binding part of the chimeric receptor; a linker derived from CD8 designated as CD8stalk; a transmembrane portion designated as CD8TM; a signal transducing unit designated as 41BB and the ζ part which is the ζ cytoplasmic tail taken from the CAR (Imai et al., 2004; Maude et al., 2018). This CAR is named in the present invention ζCAR.
**Figure 2** shows schematically embodiments of the present invention whereby each structure designated as εRKζCAR, εAAζCAR, miniεRKζCAR and miniεAAζCAR shows embodiments having the same components as shown in Figure 1. In addition to the CAR shown in Figure 1, the chimeric receptor designated as εRKζCAR contains a co-activatory domain derived from CD3ε (RK motif, sequence RKGQRDLY, SEQ ID NO:1). εAAζCAR corresponds to εRKζCAR whereby, however, the CD3ε motif is mutated which leads to lack of function (sequence AAGQRDLY, SEQ ID NO:2). The miniεRKζCAR is a modification of the εRKζCAR. miniεRKζCAR contains the CD3ε co-stimulatory domain (RK motif, sequence RKGQRDLY, SEQ ID NO:1) and lacks the_endoplasmatic reticulum retention signal having the amino acid sequence NQRRI (SEQ ID NO:3). The miniεAAζCAR is a modification of miniεRKζCAR. miniεAAζCAR contains the non-functional CD3ε co-stimulatory domain (sequence AAGQRDLY, SEQ ID NO:2)_and lacks the endoplasmic reticulum retention signal having the amino acid sequence NQRRI (SEQ ID NO:3).

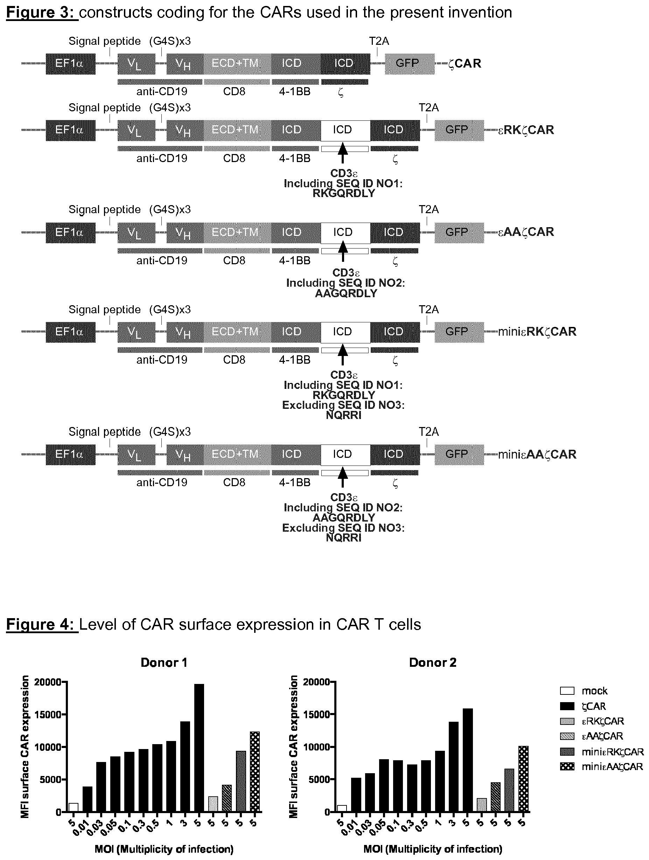

**Figure 3** schematically represents of the constructs used in the vectors for transfection. In the upper line with the designation ζCAR a construct according to the prior art is shown. After elongation factor 1α and a signal peptide, the antigen binding structure is represented by V_{L} and V_{H} coding for the FMC63 (amino acids 1-267, GenBank ID: HM852952.1) anti-CD19 region. Then the structures from the CAR ECD (extracellular domain) + TM (transmembrane domain) and ICD (intracellular domain) are present in all constructs. Furthermore, all constructs contain the ICD of ζ and the green fluorescent protein (GFP), which allows the identification of cells transfected with the constructs.
   In the next lines, the constructs according to the present invention are shown wherein a fragment of the ICD from CD3ε is present (aa amino acids 153-202, GenBank ID: NM_000733.3). The constructs codified for the following chimeric receptors: εRKζCAR, εAAζCAR, miniεRKζCAR and miniAAζCAR. εRK highlights the presence of the wild-type RK motif. εAA highlights that the RK motif is mutated (RK→AA). miniεRKζCAR and miniεAAζCAR correspond to εRKζCAR, εAAζCAR, respectively; whereby, however, the endoplasmatic reticulum retention part has been deleted (sequence NQRRI, SEQ ID NO:3).
**Figure 4** shows the results of **Experiment 1.** Expanded healthy human T cells were transfected with lentiviral vectors coding for the prior art construct shown in Figure 1 (ζCAR). A panel of different multiplicity of infection (MOI) was used to get different levels of CAR expression. εRKζCAR, εAAζCAR, miniεRKζCAR and miniεAAζCAR were also used in this experiment to a MOI of 5. The level of surface CAR expression was measured by a flow cytometric assay specifically detecting the antiCD19sc part with a fluorescent-labeled antibody. The level of surface CAR expression is proportional to the specific florescent detected and it is indicated as Mean fluorescence intensity (MFI). The results in Figure 4 show that the surface CAR expression of the chimeric receptors according to the invention is reduced when compared to the prior art construct at the same multiplicity of infection (MOI of 5). The deletion of an endoplamatic reticulum retention signal (sequence NQRRI, SEQ ID NO:3) increases substantially the surface CAR expression. The results of two independent donors of healthy human T cells are shown.

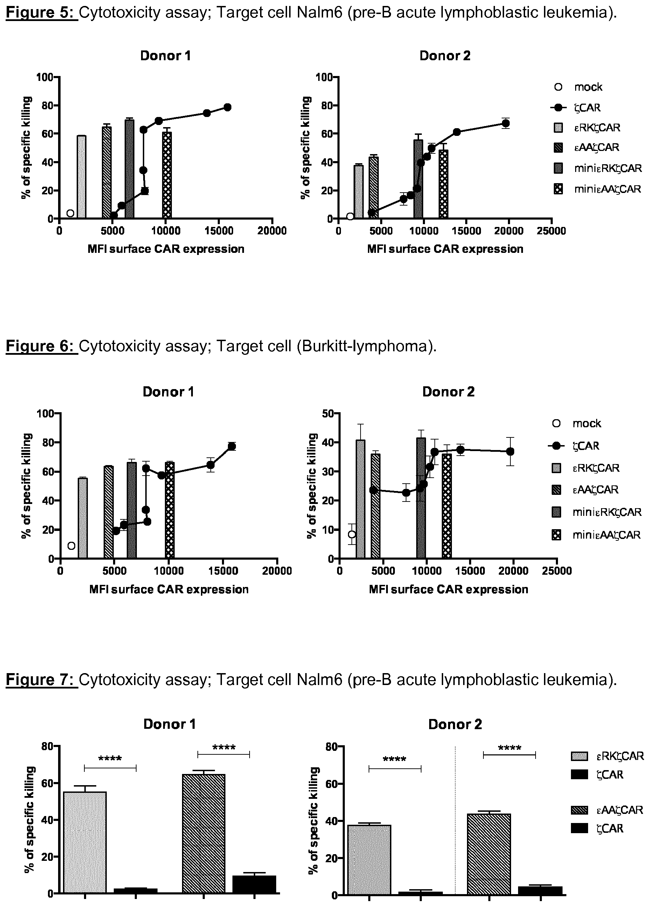

**Figure 5** shows the results of **Experiment 2**. Figure 5 shows the percent of specific killing of CD19-expressing cells (Nalm6 pre-B acute lymphoblastic leukemia) plotted against the surface CAR expression. These experiments show that the anti-tumor activity of cells expressing the embodiments εRKζCAR, εAAζCAR, miniεRKζCAR of the present invention was significantly higher than the anti-tumor activity of cells expressing the prior art ζCAR with the most similar surface levels. The anti-tumor activity of cells expressing the embodiment miniεAAζCAR of the present invention was equal or lower than the anti-tumor activity of cells expressing the prior art ζCAR with the most similar surface levels. These results demonstrated that loss-of-function mutation of the RK motif (sequence RKGQRDLY, SEQ ID NO:1 to sequence AAGQRDLY, SEQ ID NO:2) abolished the enhancement in tumor killing of the embodiments εRKζCAR and miniεRKζCAR of the present invention. The experiments were performed with expanded T cells from two different healthy donors.
**Figure 6** shows the results of **Experiment 3**. Figure 6 shows the percent of specific killing of CD19-expressing cells (DAUDI cell line, which is derived from Burkitt-lymphoma) plotted against the surface CAR expression. Data processing was done as described in Figure 5. Comparison of the CARs according to the invention to the prior art CAR (ζCAR) at the same level of surface expression demonstrates that the embodiments of the present invention εRKζCAR, εAAζCAR and miniεRKζCAR have a much higher anti-tumoral activity against DAUDI lymphoma cells. Loss-of-function mutation of the RK motif (sequence RKGQRDLY, SEQ ID NO:1 to sequence AAGQRDLY, SEQ ID NO:2) abolished the enhancement in tumor killing of the embodiments εRKζCAR and miniεRKζCAR of the present invention. The experiments were performed with expanded T cells from two different healthy donors.
**Figure 7** shows an alternative analysis of selected data derived from **Experiment 2.** The anti-tumor activity, or with other words the killing activity, of human T cells transduced with the lentiviral vector encoding for the prior art CAR (ζCAR) was compared with the killing activity of human T cells transduced with the vectors coding for εRKζCAR or εAAζCAR for the most similar level of expression. Figure 7 shows clearly that the chimeric receptors of the present invention have substantially higher anti-tumor killing activity when normalized to the same level of expression. The target cell, which was used in this experiment was Nalm6 (pre-B acute lymphoblastic leukemia).

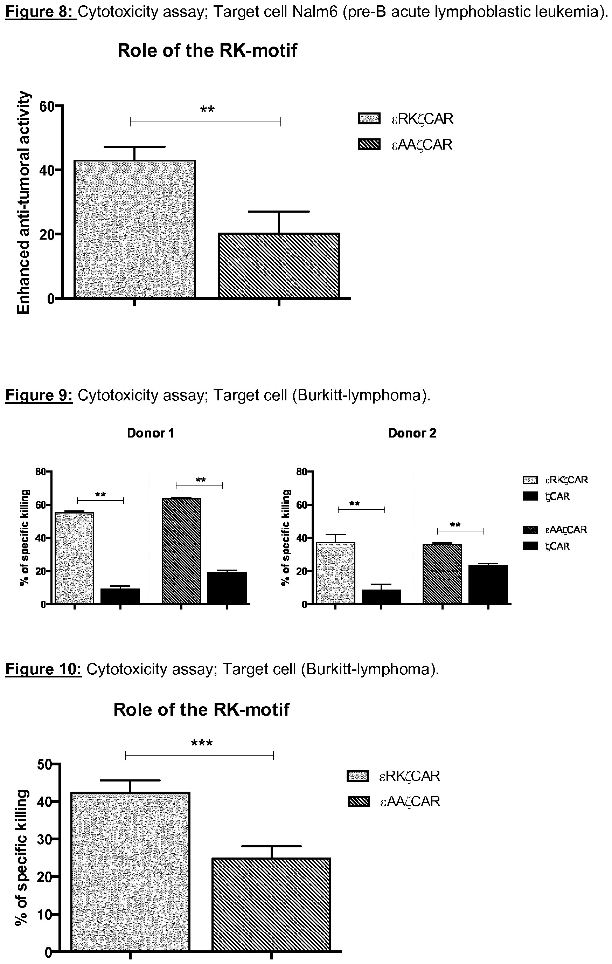

**Figure 8** pools the results of four healthy donors after independently done experiments performed as **Experiment 2.** In Figure 8, enhanced anti-tumor activity is defined as the difference between the specific killing activities of T cells expressing εRKζCAR or εAAζCAR and of T cells expressing ζCAR with the most similar surface expression. Mean values +/- SEM and p values using unpaired Student's t test are shown. When the number of expressed chimeric receptors on the cell surface is brought to a comparative level, the T cells expressing the εRKζCAR receptors show a higher anti-tumoral activity than T cells expressing the εAAζCAR. These data indicate that mutation of the RK motif strongly reduces the enhancement in tumor killing. Altogether, introducing the RK motif in a well-established CAR seems to be a good approach to increase the anti-tumor activity *in vitro* when the number of expressed chimeric receptors on the cell surface is brought to a comparative level. The target cell used in these experiments was Nalm6 (pre-B acute lymphoblastic leukemia).
**Figure 9** shows an alternative analysis of selected data derived from **Experiment 3.** The anti-tumor activity of human T cells transduced with the lentiviral vector encoding for the prior art CAR (ζCAR) was compared with the killing activity of human T cells transduced with the vectors coding for εRKζCAR or εAAζCAR for the most similar level of expression using DAUDI (Burkitt-lymphoma) cells as targets. Figure 9 shows clearly that the chimeric receptors of the present invention have substantially higher anti-tumor killing activity when normalized to the same level of expression.
**Figure 10** pools the results of five healthy donors after independently done experiments performed as **Experiment 3.** In Figure 10, enhanced anti-tumor activity is defined as in Figure 8. The target cell used in these experiments was DAUDI (Burkitt-lymphoma).

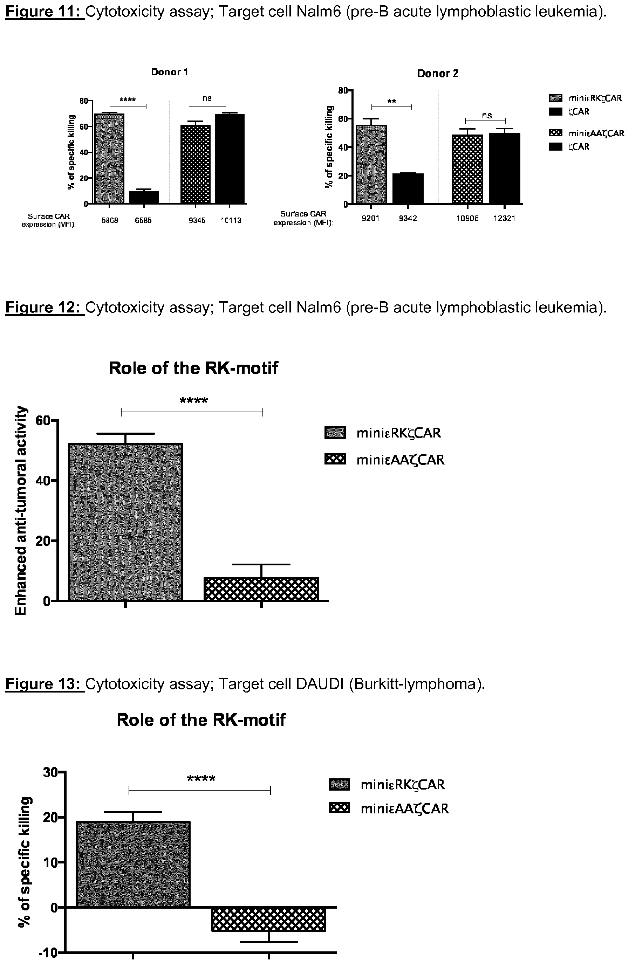

**Figure 11** shows an alternative analysis of selected data derived from **Experiment 2.** The anti-tumor killing activity of human T cells transduced with the lentiviral vector encoding for the prior art CAR (ζCAR) was compared with the killing activity of human T cells transduced with the vectors coding for miniεRKζCAR or miniεAAζCAR for the most similar level of expression. Figure 11 shows clearly that only the chimeric receptors containing the RK motif (miniεRKζCAR) of the present invention have substantially higher anti-tumor killing activity when normalized to the same level of expression. These data indicate that mutation of the RK motif strongly reduces the enhancement in tumor killing of the miniεRKζCAR presented in this invention. The target cells used in this experiment were Nalm6 (pre-B acute lymphoblastic leukemia).
**Figure 12** pools the results of two healthy donors after independently done experiments performed as **Experiment 2** and compares miniεRKζCAR with miniεAAζCAR to assay for the role of the RK motif. As above, enhanced anti-tumor activity is defined as the difference between the specific killing activities of T cells expressing miniεRKζCAR or miniεAAζCAR and of T cells expressing ζCAR with the most similar surface expression. When the number of expressed chimeric receptors on the cell surface is brought to a comparative level, the T cells expressing the miniεRKζCAR receptors show a significantly higher anti-tumoral activity than T cells expressing the miniAAζCAR. These data indicate that mutation of the RK motif strongly reduces the enhancement in tumor killing. The target cell used in these experiments was Nalm6 (pre-B acute lymphoblastic leukemia).
**Figure 13** pools the results of two healthy donors after independently done experiments performed as **Experiment 2** and compares miniεRKζCAR with miniεAAζCAR to assay for the role of the RK motif using DAUDI (Burkitt-lymphoma) as target cells. As above, enhanced anti-tumor activity is defined as the difference between the specific killing activities of T cells expressing miniεRKζCAR or miniεAAζCAR and of T cells expressing ζCAR with the most similar surface expression. When the number of expressed chimeric receptors on the cell surface is brought to a comparative level, the T cells expressing the miniεRKζCAR receptors show a significantly higher anti-tumoral activity than T cells expressing the miniAAζCAR. These data indicate that mutation of the RK motif strongly reduces the enhancement in tumor killing.

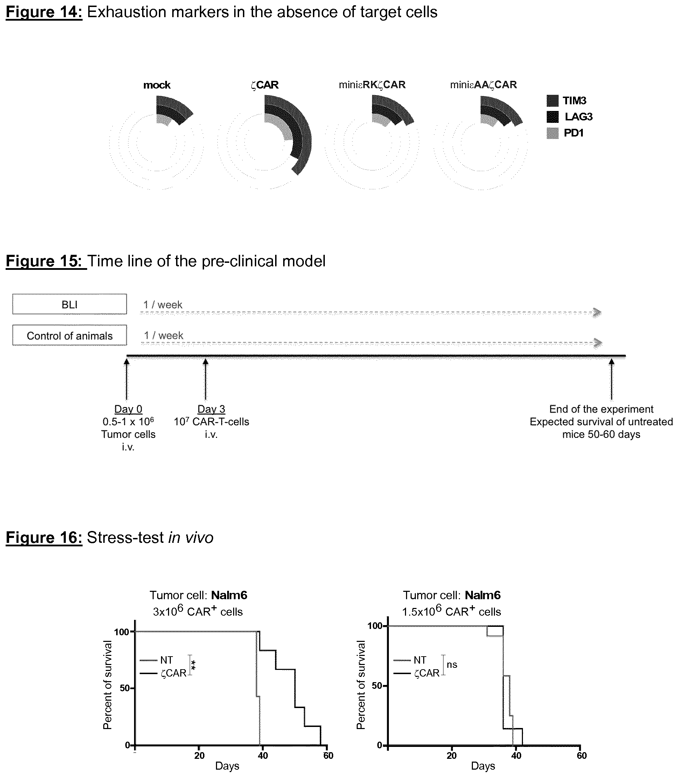

**Figure 14** shows the results of **Experiment 4.** T cell exhaustion is a major factor limiting anti-tumor responses. For this reason, we have checked up-regulation of the three exhaustion markers TIM3, LAG3 and PD1 upon expression of the ζCAR, miniεRKζCAR or miniAAζCAR. Expression of ζCAR in T cells resulted in significant up-regulation of the three exhaustion markers TIM3, LAG3 and PD1 compared to mock transduced cells in the absence of any CD19-positive cells (p<0.0001). These results are consistent with previous studies linking CAR expression levels to ligand-independent signals. In contrast, miniεRKζCAR and miniεAAζCAR transduced cells remained exhausted as the mock cells (p > 0.05).
**Figure 15** shows a schematic representation of the pre-clinical model applied to assay the anti-tumor activity of the four chimeric antigen receptors used in the present invention (Figure 2) in comparison to the well-characterized prior art ζCAR (Figure 1, Imai et al., 2004; Maude et al., 2018). The tumor cells used in this model were Nalm6 (pre-B acute lymphoblastic leukemia).
**Figure 16** plots the results of the Log-rank Mantel-Cox survival test of Nalm6-bearing mice treated 3 days after tumor cell inoculation with 3 x 10⁶ (left) or 1.5 x 10⁶ (right) ζCAR-expressing human T cells (n=4-8 mice). NT indicates non-transduced T cells. ** p<0.01. Figure 16 corresponds to **Experiment 5.**

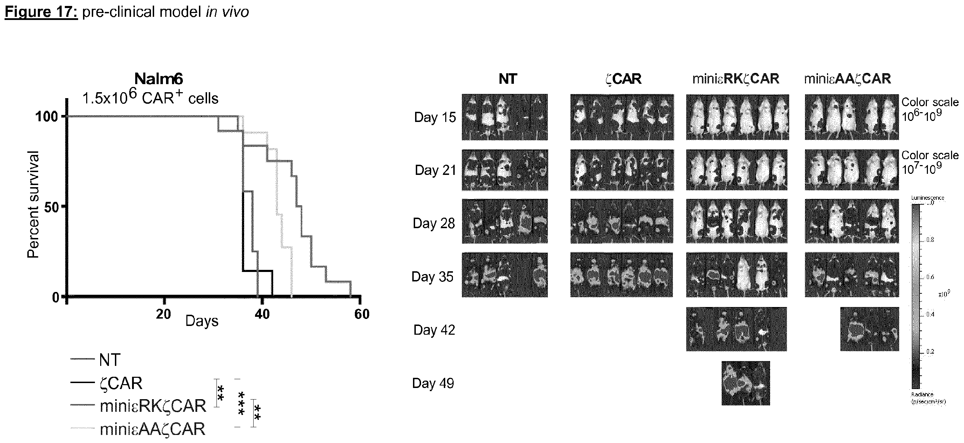

**Figure 17** plots the results of Log-rank Mantel-Cox survival test of Nalm6-bearing mice treated with 1.5x10⁶ CAR-expressing human T cells 3 days after tumor cell inoculation (n=6-12 mice pooled from two independently performed experiments). Mice were monitored by *in vivo* bioluminescence and the images shown are form one of the two experiments (right panel). NT indicates non-transduced control T cells; ζCAR indicates ζCAR-transduced T cells. miniεRKζCAR and miniεAAζCAR indicate miniεRKζCAR. transduced T cells and miniεAAζCAR-transduced T cells, respectively. ** p<0.01, *** p<0.001. Figure 17 shows the results of **Experiment 6.**
**Figure 18** shows (A-C) *In situ* proximity ligation assay (PLA) of the TCR (CD3δ) and LCK. A TCR:LCK distance smaller than 80 nm results in a red fluorescent signal. Nuclei were stained with DAPI. (D) PLA of the TCR and LCK in purified primary human T cells at 37°C for 5 min. (E) Purified human T cells were treated with PP2 and either left unstimulated, stimulated with anti-CD3ε (OKT3) or with perV at 4°C for 120 min. Statistical analysis was performed using unpaired Student's t test. Mean values +/- SEM and p values are shown. ns: not significant.
**Figure 19** (A) shows the sequences (SEQ ID NO:4-6) of the murine CD3-derived peptides used in the study. Figure 19 (B) shows a pull down (PD) assay using glutathione beads bound to GST (-) or to GST-fusion proteins containing either the LCK SH2 domain (SH2) or both LCK SH2 and SH3 domains (SH2-SH3) was performed. These beads were incubated with the indicated biotinylated peptides as shown in A ("PP" refers to doubly ITAM tyrosine phosphorylated peptides). Immunoblotting was performed using streptavidin-HRP and anti-GST antibodies (C) PD assays using GST-SH3(LCK) and GST-SH3(NCK) proteins as in B. Figure 19 (D) shows Jurkat T cell lysates that were incubated with the biotinylated CD3ε-derived peptides and streptavidin-coupled beads. Immunoblotting was performed using the indicated antibodies. Figure 19 (E) shows Jurkat T cells that were left untreated (-) or stimulated for 5 min at 37°C with anti-CD3ε antibody (+). Lysates were incubated with SH3(LCK)-coupled beads in a pull-down assay (PD). Immunoblotting was done using anti-ζ and anti-GST antibodies. Mean values +/- SEM and p values are indicated.
**Figure 20** shows an NMR titration experiment showing the change in the ¹H, ¹⁵N HSQC spectrum of free ¹⁵N-labeled SH3(LCK) (black, 50 µM) upon addition of 8- (red) or 16-fold (green) molar excess of unlabeled GST-CD3εcytoplasmic tail. sc: side chain. Black arrows connect signals coming from the same residue. Blue arrows indicate the direction of changes. Normalized chemical shift changes as a function of protein sequence (bottom panel).
**Figure 21** shows a proximity ligation assay (PLA) between the TCR (CD3δ) and LCK was performed as in Figure A. H8εsh cells expressing different Cd3ε variants were treated with PP2 and either left unstimulated (uns), stimulated with an anti-Cd3ε antibody (145-2C11) or with pervanadate (perV) at 4°C. Statistical analysis was performed between antibody-stimulated cells (grey bars) using unpaired Student's t test on pooled data of four independent experiments. Mean values +/- SEM and p values are indicated. ns: not significant.
**Figure 22** shows a backbone representation of the modeled complex formed by SH3(LCK) with the CD3ε cytoplasmic tail based on the NMR data and the current literature is displayed. Green: SH3(LCK); brown: CD3ε. Interactions are shown as dotted lines: hydrogen bonds (green), salt bridges and electrostatic interactions (black), hydrophobic, π-sigma and π-alkyl bonds (purple).
**Figure 23** (A) shows a scheme depicting the reporter for LCK activity at the TCR (ζ-Reporter). ζ was fused to LCK enzymatically inactive kinase domain (K273A) using a flexible linker. Figure 23 (B) shows the immunoprecipitation of the TCR from MA5.8 cells (murine T cells lacking ζ expression) reconstituted with ζ or with the ζ-Reporter. Cells were either left untreated (-) or stimulated (+) at 37°C with anti-Cd3ε (145-2C11). Immunoblotting was done using the indicated antibodies. Figure 23 (C) shows the quantification of the data from three independent experiments performed as in B. The relative value of phosphorylation in stimulated samples was set to 100% for each experiment. Statistical analysis compared the basal phosphorylation state of ζY142 and of Y394 both within the ζ-Reporter using Student's t test. Mean values +/- SEM and p values are shown. Figure 23 (D) shows the fold induction of Y394 phosphorylation or of the ζ ITAM tyrosine (pζY142) both within the ζ-Reporter was calculated from three experiments done as in B and statistical analysis was performed using Student's t test. Mean values +/-SEM and p values are shown.
**Figure 24** (A) shows the immunoprecipitation of phosphotyrosine-containing proteins using 4G10 (top panel). Cells were left unstimulated or stimulated with anti-Cd3ε (145-2C11) for 30 seconds. Immunoblotting was done using anti-Cd3ε (M20ε) and anti-ζ. The signal of stimulated Cd3εWT-expressing cells was set to 100% for each independent experiment. Data from four independent experiments were pooled and antibody-stimulated samples were compared to WT using unpaired Student's t test. Mean +/- SEM and p values are shown. Figure 24 (B) shows the flow cytometric analysis of calcium influx from the different H8εsh transfectants upon stimulation with 3 µg/ml anti-Cd3ε (145-2C11). Figure 24 (C) shows the IL2 production of the different H8εsh transfectants after 24 h stimulation with anti-Cd3ε (145-2C11) and anti-hCD28 was assayed by ELISA. The value of IL2 produced by Cd3εWT-cells was set to 100% for each experiment. Data from five independent experiments were pooled and the statistical analysis compared H8εsh-Cd3εWT with each of the H8εsh cell variants using unpaired Student's t test. Mean values +/- SEM and p values are shown.

**The present invention is described in more detail in the following experiments, whereby the results are presented in figures.**

In all experiments the following methods have been used:

### a) Generation of CAR constructs

The lentiviral vector pCDH-EF1-19BBζ-T2A-copGFP (coding for the ζCAR) was obtained from TCR² therapeutics. Briefly, it is a second generation CAR consisting of the scFV from murine anti-CD19 (FMC63) fused to the extracellular and transmembrane part of human CD8 alpha (amino acids 138-206), the 41BB endodomain and the ζ cytoplasmic tail. To generate the εRKζCAR and miniεRKζCAR constructs, a truncated cytoplasmic tail of human CD3ε (amino acids 153-207 and amino acids 153-202, respectively) was placed between 41BB and ζ. εAAζCAR and miniεAAζCAR constructs were generated by mutation of the εRKζCAR and miniεRKζCAR constructs, respectively, using a Site-Directed Mutagenesis kit (Agilent Technologie) according to the manufacturer's instructions.

### b) Lentivirus production

10⁷ HEK293T cells were plated on a 15 cm plate in 20 ml DMEM medium, distributed evenly, and incubated at 37°C and 7,5% CO₂. After 24 hours, medium was changed and HEK293T cells were transfected with the respective constructs and the packaging plasmids pMD2.G (envelope) and pCMVR8.74 (gag/pol) using PEI transfection. The virus-containing supernatant was harvested 24 and 48 hours after transfection and was concentrated by a 10% sucrose gradient (supplemented with 0.5 mM EDTA) centrifugation for 4 hours at 10000 rpm and 8°C. After centrifugation, the supernatant was discarded and the virus pellet was resuspended in 100µl of PBS and stored at -80°C.

### c) Primary human T cell activation, transduction, and expansion

Peripheral blood mononuclear cell (PBMCs) were purified from fresh blood of healthy donors using density centrifugation (Ficoll-Paque). PBMCs were counted, resuspended in medium supplemented with 500 U/ml recombinant human IL2 (PeproTech) and activated with plate-bound anti-CD3/CD28 antibodies (1 µg/ml). 48-72 hours after activation, the remaining PBMCs are mostly T cells (>99 %). Primary human T cells were then lentivirally transduced using spin infection in the presence of 5 µg/ml protamine sulfate (Sigma), 1000 U/ml IL2 with a MOI of 5 (at least otherwise indicated). Transduced T cells were checked for CAR expression 5-7 days after transduction using a biotinylated primary goat antimouse F(ab')² antibody (Invitrogen) followed by streptavidin-APC (Biolegend). Cells were cultured in medium supplemented with 100 U/ml IL2 for a maximum of 7 days after transduction before used for killing assays.

### d) Cytotoxicity assay

Luciferase-expressing tumor cells (Nalm6 or DAUDI as indicated) were plated at a concentration of 1x10⁵ cells/ml in 96-well flat bottom plates in triplicates. 75 µg/ml D-firefly luciferin potassium salt (Biosynth) was added to the tumor cells and Bioluminescence (BLI) was measured in the luminometer (Tecan infinity M200 Pro) to establish the BLI baseline. Right after, CAR-T cells were added at 5:1 effector-to-target (E:T) ratio and incubated for 8 or 24 hours (as indicated) at 37°C. BLI was measured as relative light units (RLU). RLU signals from cells treated with 1% Triton X-100 indicates maximal cell death. RLU signals from tumor cells without CAR-T cells determine spontaneous cell death. Percent specific lysis was calculated with the following formula: % specific lysis = 100x (average spontaneous death RLU- test RLU)/ (average spontaneous death RLU- average maximal death RLU).

By using the above shortly described general methods the following experiments were performed:

### Experiment 1:

Expanded human T cells from healthy donors were transduced with a lentiviral vector coding for the ζCAR at different multiplicities of infection (MOI) to obtain different levels of expression of the ζCAR on the cell surface. Increasing the MOI elevated the level of ζCAR expression as tested by flow cytometry using fluorescent-labelled antibodies against the extracellular part of ζCAR. Expanded human T cells from healthy donors were also transduced with lentiviral vectors coding for the embodiments of the present invention εRKζCAR, εAAζCAR, miniεRKζCAR and miniεAAζCAR at MOI 5. The results are shown in **Figure 4****.** By choosing the appropriate MOIs for the ζCAR, the prior art ζCAR can be compared with the constructs of the invention, which were expressed to a lower level.

### Experiment 2:

The anti-tumor activity (killing activity) of human T cells transduced with the lentiviral vector coding for the ζCAR at different multiplicities of infection (MOI) correlates with the level of surface expression of ζCAR. Donors were healthy volunteers. The target cells were Nalm6 pre-B acute lymphoblastic leukemia. Expanded human T cells transduced with the lentiviral vector were incubated with the target cells at 5:1 effector-to-target (E:T) ratio and incubated for 8 at 37°C. The specific anti-tumor activity was calculated as indicated in the methods section (Cytotoxicity assay). Comparison of the CARs according to the invention to the prior art CAR (ζCAR) at the same level of surface expression demonstrates that the embodiments of the present invention εRKζCAR, εAAζCAR and miniεRKζCAR have a higher anti-tumoral activity. The results are shown in **Figures 5, 7****,****8****,** **11 and 12****.**

### Experiment 3:

The anti-tumor activity (killing activity) of human T cells transduced with the lentiviral vector coding for the ζCAR at different multiplicities of infection (MOI) correlates with the level of surface expression of ζCAR. Donors were healthy volunteers. The target cells were DAUDI cells, which were derived from a Burkitt-lymphoma. Expanded human T cells transduced with the lentiviral vector were incubated with the target cells at 5:1 effector-to-target (E:T) ratio and incubated for 24 hours at 37°C. The specific anti-tumor activity was calculated as indicated in the methods section (Cytotoxicity assay). Comparison of the CARs according to the invention to the prior art CAR (ζCAR) at the same level of surface expression demonstrates that the embodiments of the present invention εRKζCAR, εAAζCAR and miniεRKζCAR have a much higher anti-tumoral activity. The results are shown in **Figures 6****,** **9****,** **10** **and** **13****.**

### Experiment 4:

T cell exhaustion is a major factor limiting anti-tumor responses. For this reason, we have checked up-regulation of the three exhaustion markers TIM3, LAG3 and PD1 upon expression of the ζCAR, miniεRKζCAR or miniAAζCAR. To this end, 1.2 x 10⁵ CAR T cells expressing the indicated constructs were analyzed by flow cytometry for the surface expression of exhaustion markers. The analysis was performed 8 days after transduction. The following antibodies were using for the detection of exhaustion markers in CAR T cells: Alexa Fluor 647-labeled anti-human CD223 (LAG-3), PE/Cy7-labeled anti-human CD366 (Tim-3) and biotin-conjugated anti-human CD279 (PD-1). The results are shown in **Figure 14****.**

### Experiment 5:

In order to test the anti-tumor activity of CARs *in vivo,* we performed a "stress test" in a pre-clinical mouse model using Nalm6 as previously described (Zhao et al., 2015). Briefly, Nalm6-tumor bearing mice were injected with CAR-T cell doses that are purposefully lowered to levels where well-established CAR therapy *in vivo* starts to fail. In our hands, this point was reached with a dose of 1.5 x 10⁶ ζCAR-positive cells. Mice were controlled weekly and the survival proportions plotted. The results are shown in **Figure 16****.**

### Experiment 6:

The "stress test" pre-clinical mouse model using Nalm6 was performed as in Experiment 5. Nalm6-tumor bearing mice were injected with 1.5 x 10⁶ CAR-positive cells. T cells expressing ζCAR,_miniεRKζCAR and miniεAAζCAR were used. Mice were controlled weekly and the survival proportions plotted. Mice were subjected to *in vivo* bioluminescence imaging (BLI) for firefly luciferase weekly. To this end, mice were injected i.p. with 200 mg/kg luciferin (Sigma, Germany) dissolved in distillated water. The mice were imaged in the bioluminescence camera 10 min after injection, with optimal settings for luminescent exposure. The results are shown in **Figure 17****.**

## Claims

1. A chimeric antigen receptor comprising at least the following components:
a) a peptidic structure capable of binding to a ligand;
b) an extracellular spacing structure;
c) a transmembrane domain;
d) none or at least one co-stimulatory domain; and
e) at least one TCR-derived activatory domain
**characterized in that** the TCR-derived activatory domain comprises the amino acid sequence RKGQRDLY (SEQ ID NO:1) which is capable to bind the lymphocyte specific Src kinase.

2. Chimeric antigen receptor according to claim 1 **characterized in that** the TCR-derived activatory domain comprising the amino acid sequence RKGQRDLY (SEQ ID NO:1) is derived from the cytoplasmatic tail of the CD3ε receptor.

3. Chimeric antigen receptor according to claim 1 or 2 **characterized in that** the sequence RKGQRDLY is capable of binding the lymphocyte specific Src kinase.

4. Chimeric antigen receptor according to claims 1 to 3 **characterized in that** the peptidic structure is capable of binding a ligand (a) is an antigen binding structure.

5. Chimeric antigen receptor according to claim 4 **characterized in that** the antigen binding moiety is a single chain fragment (scFv), a nanobody, a naturally occurring ligand or an aptamer.

6. Chimeric antigen receptor according to any of claims 1 to 5 **characterized in that** the it includes the TCR-derived activatory domain comprising the amino acid sequence RKGQRDLY (SEQ ID NO:1) but excludes the endoplasmatic reticulum retention signal naturally found in CD3ε amino acid sequence NQRRI (SEQ ID NO:3).

7. Chimeric antigen receptor according to any of claims 1 to 6 **characterized in that** it comprises two TCR-derived activatory domains.

8. Vector comprising the genetic information coding for a chimeric antigen receptor according to any of claims 1-7.

9. Vector according to claim 8 **characterized in that** it is a lentiviral vector.

10. Process for transfecting peripheral blood with a vector according to claims 8 or 9 and transfected cells are harvested and concentrated.

11. Process according to claim 10 wherein T cells are collected and isolated ex vivo and thereafter transfected with a vector according to claims 8 or 9.

12. Process according to claim 11 **characterized in that** the T cells transfected with a chimeric receptor are grown and expanded in vitro.

13. Chimeric antigen receptor according to any of claims 1 to 8 for use in the treatment of a disease, in particular in the treatment of cancer.

14. Chimeric antigen receptor according to any of clams 1 to 8 encoded by a vector for the use according to claim 12.

15. T cell comprising a chimeric antigen receptor according to any of claims 1 to 8 for use in the treatment of cancer.
